# EUROPEAN PATENT APPLICATION

(11) **EP 4 424 247 A1**
(43) Date of publication of application: **04.09.2024**
(21) Application number: 23159740.2
(22) Date of filing: 02.03.2023
(51) Int. Cl.: A61B 17/04, A61B 17/06, A61B 17/062, A61F 2/02

(54) **SURGICAL DEVICE HAVING A FELTING NEEDLE WITH A FLEXIBLE TIP**

(71) Applicant: ZuriMED Technologies AG, 8008 Zürich (CH)
(72) Inventor: BRUN, Sascha, 8006 Zurich (CH); BANZET, Pol, 8006 Zürich (CH); LI, Xiang, 8610 Uster (CH); BACHMANN, Elias, 8049 Zurich (CH); MÖHL, Andrea Gabriela, 5600 Lenzburg (CH); SENN, Ronja, 5436 Würenlos (CH)
(74) Representative: Vossius & Partner Patentanwälte Rechtsanwälte mbB

(57) **Abstract**

The invention relates to a surgical device (1) for attaching a non-woven textile (34; 44) to animal soft tissue (35; 45). The non-woven textile is attached to the human or animal tissue by carrying individual fibers of the non-woven textile into the human or animal soft tissue. The device comprises a holder (3, 5) and a felting needle (2). The holder holds the felting needle. The felting needle is movable reciprocally between a first position and a second position relatively to the holder. The felting needle has a longitudinal axis, a proximal end, and a distal end. The felting needle is configured to push individual fibers of the non-woven textile into the human or animal soft tissue. At least a tip portion of the felting needle is flexible. The tip portion has at least a length between the first and second position, and the tip portion is configured to deflect upon hitting a hard surface.

## Description

The present application relates to a surgical device for attaching a non-woven textile to human or animal soft tissue by carrying individual fibers of the non-woven textile into the human or animal soft tissue. The invention also relates to a method of attaching a non-woven textile to human or animal soft tissue by carrying individual fibers of the non-woven textile into the human or animal soft tissue.

The present applicant recently introduced the Surgical FiberLock Technology (see e.g., PCT/CH2019/000015) as a method and technology for implanting a dedicated biocompatible implant made of non-woven microfibers using a dedicated device. This innovative technology allows for the distribution of microfibers throughout soft tissue to repair it and augment its repairs, while also minimizing the occurrence of suture cut through, which is a common issue resulting in revision of repairs using traditional suturing. Additionally, this technology has the potential to reinforce and repair severely damaged or weak tissue that could not be repaired with traditional suturing.

The Surgical FiberLock Technology implantation process involves the utilization of a high frequency reciprocating needle. The needle penetrates both, a non-woven implant and human or animal soft tissue, and is designed with features that allow it to carry the implant fibers into the soft tissue. As a result, the implant becomes securely and evenly anchored in the tissue.

As previously disclosed, the Surgical FiberLock Technology requires manual handling of the instrument while the needle moves at high speeds, which creates a risk of needle damage or breakage in the event of accidental contact with a hard surface, such as bone or a stainless-steel surgical tool. To mitigate this risk, a prior invention introduced a buckling wire acting as a mechanical clutch that buckles when a dangerous load is reached (see EP 21211467.2). While this invention reduced most of the risks, residual risks still exist, such as the risk of needle breakage due to strong bending of the needle body in the event of a damaged device with a needle locked in its extended position, or in the case of hitting a specifically curved hard surface.

It is the objective technical problem to overcome one or more of the above disadvantages of the prior art. In particular, it is the objective technical problem to provide a surgical device for attaching a non-woven textile to human or animal soft tissue by carrying individual fibers of the non-woven textile into the human or animal soft tissue that prevents damage to the felting needle.

A first aspect of the invention relates to a surgical device for attaching a non-woven textile to human or animal soft tissue. The non-woven textile is attached to the human or animal tissue by carrying individual fibers of the non-woven textile into the human or animal soft tissue. The device comprises a holder and a felting needle. The holder holds the felting needle. The felting needle is movable reciprocally between a first position and a second position relatively to the holder. The felting needle has a longitudinal axis, a proximal end, and a distal end. The felting needle is configured to push individual fibers of the non-woven textile into the human or animal soft tissue. At least a tip portion of the felting needle may be flexible. The tip portion has at least a length between the first and second positions, and the tip portion is configured to deflect upon hitting a hard surface.

The present invention introduces a needle that is compatible with the Surgical FiberLock Technology and has a flexible tip portion. For example, the tip portion is made of a flexible material. Alternatively or additionally, the dimensions and the geometry of the tip portion may be appropriately chosen such that the needle tip is flexible. This enables the tip portion of the needle to deflect upon contact with a hard surface.

Felting as used herein may be understood as carrying, e.g., pushing, a single or a few fibers ("individual fibers") of the non-woven textile into the human or animal soft tissue with a felting needle. This may result in an intertwining and/or entangling of tissue fibers and implant fibers. Additionally or alternatively the fibers might stick within the soft tissue, i.e. a friction based attachment

The reciprocal motion may be understood as a back-and-forth motion. For example, the needle is moved along a line or rotated around an axis about an angle. The non-woven material may be or form part of an implant. The implant may be provided with the surgical device. Example materials and example implants are shown for example in PCT/CH2019/000015, PCT/EP2020/081887, PCT/EP2020/081891, and PCT/EP2020/081881. The implants may comprise a drug as described in EP 22177290.8.

The holder may have a tubular member that guides that felting needle along the reciprocal movement. The surgical device may also include a driving member for moving the felting needle reciprocity between a first and second position. A distance between the first and second positions may be 1 to 30 mm, more preferably 6 to 15 mm. In particular examples, the amplitude is 6.5 mm 8.5 mm, or 12 mm. Thus, the tip portion may have a length between 1 and 30 mm more preferably 6 to 15 mm, preferably 8.5 or 12 mm. The felting needle may have a length of at least the thickness of the non-woven textile plus 2 mm. In some embodiments, the tip portion may have a length of at least 4, 6, 8, or 10 mm. The motor may actuate the needle with a frequency of 1-200 Hz, preferably 10-100 Hz, most preferably 20-80 Hz and in one embodiment 40 Hz.

In a preferred embodiment, the felting needle is made of a flexible material. The flexible material is preferably a superelastic material such as nitinol. Using a flexible material provides for a particularly simple implementation and manufacture of the felting needle. Alternatively, the tip portion may have reduced dimensions and a different shape.

Superelastic materials such as nitinol are particularly preferred. Superelastic materials, also known as shape memory alloys (SMAs), are a type of material that can undergo large deformations and return to their original shape upon the removal of stress or strain. This unique behavior may be a result of a reversible phase transformation that occurs within the material. Superelastic materials are typically made from alloys of metals such as nickel, titanium, and copper. When a superelastic material is subjected to stress or strain, the atoms within the material may rearrange themselves into a new crystal structure, which allows the material to undergo large deformations without breaking. This deformation can be caused by mechanical forces, such as bending or twisting. Once the stress or strain is removed, the material undergoes a reversible transformation back to its original shape. This behavior may be because the new crystal structure is unstable, and the atoms will naturally return to their original positions as the material relaxes.

In the present application this behavior is used to prevent damage to the tip portion of the needle. Instead of breaking, the needle tip simply deflects and returns to its original shape once the stress is removed.

The first position and the second position may define the endpoints of the reciprocal motion. In the first position, the felting needle may be in its most distal position and in the second position, the felting needle may be in the most proximal position of the reciprocal motion.

In a preferred embodiment, the surgical device additionally includes the wire that is connected to the felting needle. The wire is configured to transfer a reciprocal motion to the felting needle. The wire and the felting needle are integrally formed. The surgical device may comprise such a wire to transfer the reciprocal motion to the felting needle. One end of the wire may be connected to the felting needle, the other end of the wire may be connected, directly or indirectly, to an actuator, e.g. a motor. Since felting needle and wire are integrally formed, they can be provided as one piece and be manufactured from a single piece of wire.

In a preferred embodiment, the holder comprises a tubular member. The felting needle may be held within the tubular member in a transport position and/or in a second position. The felting needle is at least partially expelled from the tubular member through a distal tubular member opening in the tubular member in the first position. Distal and proximal as used herein are used to describe directions with respect to an operator of the surgical device. Proximal defines directions that point generally towards an operator of the surgical device, and distal means a direction pointing away from the operator of the surgical device. The tubular member protects the felting needle during insertion into a human or animal body and from any mechanical contact.

In a preferred embodiment, the tip portion has a bent resting shape. The felting needle is loaded in the tubular member and configured to assume a bent shape upon being expelled from the tubular member. While the felting needle is held within the tubular member, the felting needle is forced to assume substantially the shape of the tubular member. Upon being expelled, the felting needle (or at least parts thereof) can freely bend and assume its original shape. This allows accessing difficult to reach parts of the body, in particular in minimally invasive operations. For example, tissues that are arranged to the sides of the tubular member may be felted. The felting needle may form a pre-stressed spring when in the tubular member. Thus, difficult-to-reach areas of soft tissue, such as when the felting needle can only access the tissue at a low angle, become accessible.

In another embodiment, the felting needle may be forced to assume a bent shape using the tubular member. The tubular member could be bent actively or passively. For example, the surgical device may comprise a bending mechanism (e.g. with strings) for bending the tubular member and thus the felting needle. The tubular member could also have a curved resting shape, while at the same time being flexible. In this embodiment, the tubular member could be forced into a straight shape by the user or a mechanism and assume its bent resting shape upon being released.

In a preferred embodiment, the tip portion extends or points in a direction at an angle of 30° or more with respect to the tubular member. Further preferred, the tip portion extends in a direction at an angle of 45°, 60°, 70°, 80° or 90° or more. These angles are examples of the bent needle.

In a preferred embodiment, an axis of the distal tubular member opening extends in a direction at an angle of 45°, 60°, 70°, 80° or 90° or more to the distal tubular member. As mentioned above, the distal tubular member opening may force the felting needle to be angled with respect to the tubular member. If the opening extends in an angled direction, this forces needle to point in this direction, which allows for felting soft tissues at an angle.

In a preferred embodiment, the device additionally comprises a clamp for the non-woven textile. The clamp may comprise a first arm and a second arm. In some embodiments, the second arm may be formed by the tubular member. The first arm and the second arm may be pivotably attached to each other, e.g. at a pivot point. The clamp may include a jaw for gripping the non-woven textile. The arms may be biased towards each other, e.g., using a spring. Such a clamp may also be used independently of a flexible tip portion.

The clamp may extend at least partially in parallel to the holder such that the non-woven textile can be clamped between the clamp and the holder. This provides for a particularly simple implementation, as only one additional element (e.g. a clamp arm) is needed. The additional clamp arm may be straight or have a curved shape.

In a preferred embodiment, the felting needle is directed towards the clamp upon being expelled. Thus, the felting needle is directed towards the non-woven textile that is held by the clamp. As a result, the implant comprising the non-woven textile can be preloaded in the surgical device. The implant can then be positioned within the human or animal body and immediately be felted to the corresponding soft tissue. This allows for a simple implantation technique.

In a preferred embodiment, the distal tubular member opening is in a sidewall of the tubular member. The distal tubular member opening being in a sidewall may force the felting needle to exit the tubular member at an angle to the tubular member. Thus, the felting needle is directed at an angle to the tubular member. This allows felting soft tissues that are adjacent to the tubular member and may otherwise be difficult to access with a felting needle. In some embodiments, a distal end of the tubular member may be closed. This may allow felting non-woven textiles that are clamped between the tubular member and an arm of the clamp.

In a preferred embodiment, the distal tubular member opening is in the distal end of the tubular member. Additionally, the felting needle may have a bent resting shape. It is particularly preferred that the felting needle is made of a super elastic material in this embodiment. In these cases, the tubular member may force the at least one felting needle to conform with the shape of the tubular member. Upon reaching the distal tubular member opening exiting and the tubular member the felting needle assumes its bent resting shape. This may also allow felting non-woven textiles that are clamped between the tubular member and an arm of the clamp or generally at the side to the tubular member. The felting needle may be considered to be a pre-loaded leaf spring with a single leaf.

In a preferred embodiment, the felting needle is made of a flexible material and wherein the flexible material is nitinol, stainless steel, titanium, and/or a polymer such as PEEK, PET, or POM. These materials can be used to design a flexible needle tip. In the case of nitinol, the needle tip becomes flexible due to the material properties. Needle tip portions that are made out of the other materials can have reduced dimensions and/or a different geometry, such as cutouts in the tip portion, that allows for an increased flexibility in the tip portion. For example, the tip portion may be tempered or otherwise have an increased flexibility. In another example, the tip portion forms a tightly wound spring.

In a preferred embodiment, the surgical device additionally comprises an implant including the non-woven textile. The non-woven textile is held by the clamp or between the clamp and the tubular member.

A further aspect of the present invention relates to a method of operating a surgical device as described above. The method comprises the following steps:
- Providing a surgical device according to one of the preceding claims;
- Actuating the felting needle in a reciprocal motion;
- Contacting a hard surface with the felting needle; and
- Deflecting the felting needle when hitting the hard surface due to the felting needle having the distal end portion being flexible.

In a preferred embodiment, the needle pushes the fibers of the non-woven textile into the human or animal soft tissue.

One particular application for the surgical devices disclosed above is the repair of a rotator cuff tendon. The rotator cuff tendon is close to bone tissue and thus there is a high risk of needle damage during repair. Further, the conventional access points in a minimally invasive operation result in an access parallel to the rotator cuff tendon. Thus, the embodiments in which the needle is angled and/or is bent or forced to bend are particularly advantageous. Similarly, soft tissue, e.g. tendons, of the knee can now be accessed in a minimally invasive procedure. Previously such tendons were inaccessible for a fast-moving felting needle in a minimally invasive operation.

Non-limiting embodiments of the invention are described, by way of example only, in the accompanying drawings, in which:
- Figure 1:: shows a surgical device as known in the art.
- Figures 2A to 2D:: show a sequence of events of a surgical device according to the invention with a flexible needle tip that deflects upon hitting a hard surface.
- Figures 3A to 3D:: show a surgical device according to the invention which holds a nonwoven textile that is positioned at the side of a tubular member.
- Figures 4A to 4D:: show a further embodiment of a surgical device according to the invention, which felts a non-woven textile that is positioned to the side of a tubular member.
- Figure 5A to 5E:: show a further embodiment of a surgical device that additionally includes a clamp.

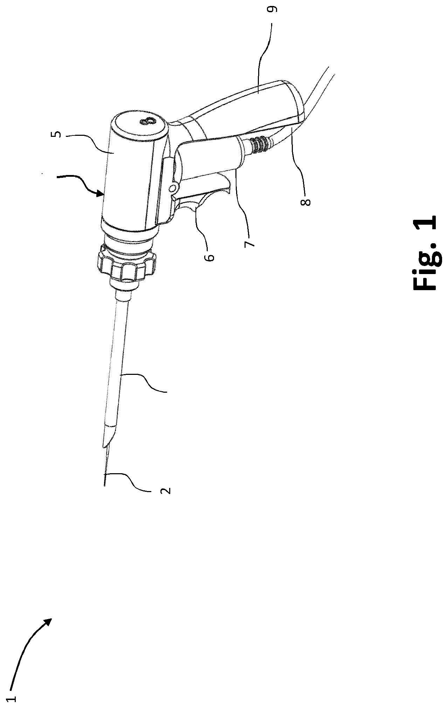

Figure 1 shows a perspective view of a surgical device 1 as disclosed in WO 2022/100833 (ZURIMED TECHNOLOGIES AG). The surgical device 1 includes a holder that comprises a housing 5 and a tubular member 3 extending from the housing 5. A felting needle 2 is arranged within the tubular member 3 and extends from the end of the tubular member 3. The felting needle 2 can be moved reciprocally forwards and backwards along its axis and the axis of the tubular member 3. The surgical device 1 includes a handle 9 with a trigger lever 6. Further, the surgical device 1 is connected to an energy source with a power cord 8 that connects to the surgical device 1. An actuator - here electrical motor 7 - is arranged within the housing 5 of the surgical device 1. The motor 7 drives the reciprocal motion of the needle 2. When a user pulls the trigger lever 6, the electrical motor 7 moves the felting needle 2. The felting needle moves back and forth along its longitudinal axis with a frequency of about 40 Hz with an amplitude of 12 mm in this particular embodiment (other frequencies and amplitudes are possible). Such a surgical device is disclosed in detail in WO 2022/100833.

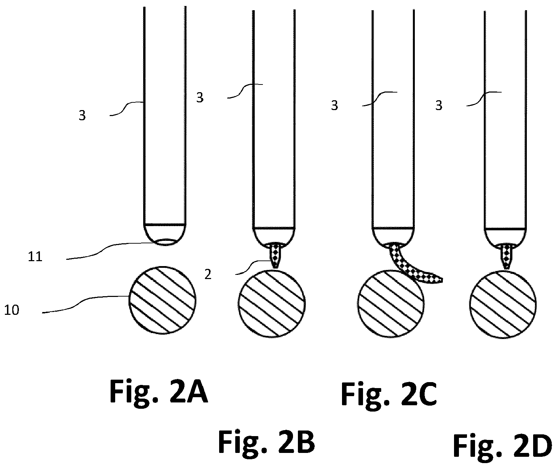

Figures 2A to 2D show a distal portion of the tubular member 3 of the surgical device 1 in detail. In figures 2A to 2D, a sequence of events is shown. In the depiction to the very left (figure 2A) the felting needle is in its retracted position. A hard tissue is arranged distally in front of a distal end of the tubular member 3. The felting needle 2 is in a second position in figure 2A. In the second position, the felting needle may be fully retracted within the tubular member 3. When the felting needle 2 is moved, in particular during the reciprocal motion, the felting needle exits a distal tubular member opening 11 (see figure 2B). The felting needle 2 is made of nitinol or any other super elastic material. During operation, the felting needle moves at high speeds and high frequencies. Thus, an operator may accidentally move the felting needle 2 too close to a hard tissue 10 such as bone. Upon hitting the hard tissue (see figure 2C) a tip portion of the felting needle 2 deflects without breaking. Upon retraction (see depiction to the right) the felting needle is retracted back into the tubular member 3. As a result of the material properties of nitinol, the felting needle 2 can hit a hard surface such as bone without breaking or causing damage to the device.

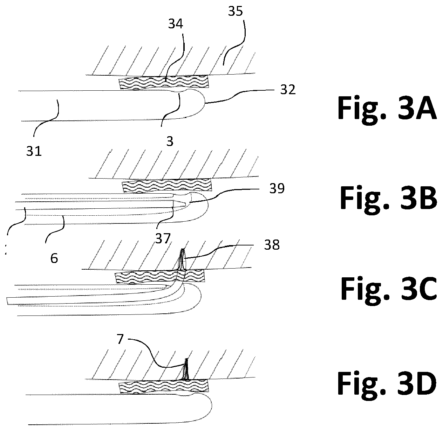

Figures 3A to 3D show a distal portion of another tubular member 31 according to the invention. As can be seen in figure 3A, the tubular member includes a distal end 32. The distal end 32 of the tubular member 31 is closed. Rather, the tubular member 31 includes a tubular member opening 33 that is in a sidewall of the tubular member 31. The tubular member opening 33 is positioned adjacent a non-woven textile 34 and a soft tissue 35. Figure 3B shows a cross-section of the tubular member 31. As can be seen, the tubular member includes an inner channel 36. The felting needle 2 is arranged within the inner channel 36. The inner channel 36 leads towards the tubular member opening 33 and is rounded at its distal end such that it guides the felting needle 2 towards the tubular member opening 33. The inner channel 36 guides the felting needle 2 with a rounded shape 39 that forces a distal tip portion 37 of the needle 2 to point in a direction at an angle to, here perpendicularly to, the tubular member 31.

Upon exiting the tubular member opening 33, the felting needle 2 first penetrates the non-woven textile 34, catches individual fibers of the non-woven textile 34 and pushes fibers of the non-woven textile 34 into the soft tissue 35 (see figure 3C). Then, the felting needle is retracted into the tubular member 31 (see figure 3D). As can be seen in figure 3D, fibers 38 remain within the soft tissue, which forms a bond between the non-woven textile 34 and the soft tissue 35. This process is repeated with the frequency of about 40 Hz until a sufficiently strong attachment is reached. The tubular member 31 may be moved along the non-woven textile 34 and the soft tissue 35 to attach non-woven textile 34 and soft tissue 35 over a large area.

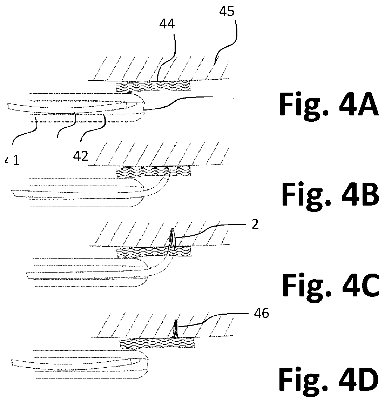

Figures 4A to 4D show a distal portion of another tubular member 41 according to the invention. Figure 4A shows a cross-section of a distal end portion of the tubular member 41. The tubular member 41 includes an inner channel 42 and a tubular member opening 43 in the distal end of the tubular member 41. The felting needle 2 is arranged within the inner channel of the tubular member 41 and has a curved resting shape. In the resting shape the felting needle 2 may be rounded. In the shown embodiment, a tip portion of the felting needle 2 may points in a direction of 90° with respect to the rest of the needle. Other angles may be chosen based on the application. The tubular member 41 forces the felting needle 2 into a substantially straight form, i.e. the substantially straight form of the inner channel 42 of the tubular member 41. Thus, the felting needle is pre-stressed within the tubular member. When the felting needle 2 is expelled from the tubular member opening 43, the felting needle 2 assumes its bent resting shape as can be seen from figure 4B. Figures 4A to 4D also show a non-woven textile 44 that is arranged on a soft tissue 45. The felting needle 2 pushes through the non-woven textile 44 and into the soft tissue 45. When pushing through the non-woven textile 44 the felting needle 2 catches individual fibers 46 of the non-woven textile 44 and pushes these fibers into the soft tissue 45. These fibers remain within the soft tissue 45 as can be seen in figure 4D.

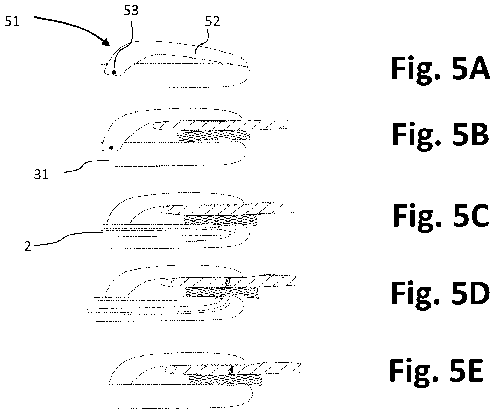

The embodiment shown in figures 5A to 5E is similar to the embodiment of figures 3A to figure 3D. In addition, the surgical device 1 comprises a clamp 51. The clamp 51 includes a clamp arm 52. One end of the clamp arm 52 is connected at a pivot point 53 to the tubular member 31. Thereby, the clamp arm 52 is pivotably connected to the tubular member 31. The clamp arm 52 may include a spring (not shown) that biases the clamp arm 52 towards the tubular member 31. The clamp arm 52 is arc shaped. The arc shape concentrates the clamping force onto a distal end of the clamp arm 52 to increase the hold of the clamp 51. The distal end of the clamp arm 52 and the distal end portion of the tubular member 31 thereby form jaws that can hold the soft tissue 35 and the non-woven textile 34 together and in place. When the felting needle 2 is moved back and forth, the soft tissue 35 and the non-woven textile 34 are securely kept in place.

## Claims

1. A surgical device (1) for attaching a non-woven textile (34, 44) to human or animal soft tissue by carrying individual fibers of the non-woven textile (34, 44) into the human or animal soft tissue, the device comprising:
a holder and a felting needle (2); the felting needle (2) being held by the holder, wherein the felting needle (2) is movable reciprocally between a first position and a second position relatively to the holder,
wherein the felting needle (2) has a longitudinal axis, a proximal end, and a distal end (32), the felting needle (2) being configured for pushing individual fibers of the non-woven textile (34, 44) into the human or animal soft tissue,
**characterized in that** at least a tip portion (37) of the felting needle (2) is flexible, wherein the tip portion (37) has at least a length between the first and the second position, and wherein the tip portion (37) is configured to deflect upon hitting a hard surface.

2. Surgical device according to claim 1, wherein the felting needle (2) is made of a flexible material, preferably a superelastic material.

3. Surgical device according to one of the preceding claims, wherein the device additionally includes a wire that is connected to the felting needle (2), wherein the wire is configured to transfer a reciprocal motion to the felting needle (2) and wherein the wire and the felting needle (2) are integrally formed.

4. Surgical device according to one of the preceding claims, wherein the holder comprises a tubular member (3, 31, 41), wherein the felting needle (2) is held within the tubular member (3, 31, 41) in a transporting position and wherein the felting needle (2) at least partially expelled from the tubular member (3, 31, 41) through a distal tubular member opening (11) in the tubular member (3, 31, 41) in the first position.

5. Surgical device according to one of the preceding claims, wherein the tip portion (37) has a bent resting shape, wherein the felting needle (2) is loaded in the tubular member (3, 31, 41) and configured to assume a bent shape upon being expelled from the tubular member (3, 31, 41).

6. Surgical device according to claim 3, wherein the tip portion (37) extends or points in a direction at an angle of 30° or more with respect to the tubular member (3, 31, 41), preferably at an angle of 45°, 60°, 70°, or 90° or more.

7. Surgical device according to one of the preceding claims, wherein the device additionally comprises a clamp (51) for the non-woven textile (34, 44).

8. Surgical device according to claim 5, wherein the clamp (51) extends at least partially in parallel to the holder such that the non-woven textile (34, 44) can be clamped between the clamp (51) and the holder.

9. Surgical device according to one of claims 3 to 4 and to claim 5 or 6, wherein the felting needle (2) is directed towards the clamp (51) upon being expelled.

10. Surgical device according to one of the preceding claims, wherein the distal tubular member opening (11) is in a sidewall of the tubular member (3, 31, 41).

11. Surgical device according to one of the preceding claims, wherein the distal tubular member opening (11) is in the distal end (32) of the tubular member (3, 31, 41).

12. Surgical device according to one of the preceding claims, wherein the felting needle (2) is made of a flexible material and wherein the flexible material is nitinol, stainless steel, titanium, aluminum, and polymers such as PEEK, PET, or POM.

13. Surgical device according to one of the preceding claims, additionally comprising an implant including a non-woven textile (34, 44), wherein the non-woven textile (34, 44) is held by the clamp (51) or between the clamp (51) and the tubular member (3, 31, 41).

14. Method of operating a surgical device (1) according to one of the preceding claims, the method comprising:
- Providing a surgical device (1) according to one of the preceding claims;
- Actuating the felting needle (2) in a reciprocal motion;
- Contacting a hard surface with the felting needle (2); and
- Deflecting the felting needle (2) when hitting the hard surface due to the felting needle (2) having the distal end portion that is flexible.

15. Method of felting a non-woven textile (34, 44) to human or animal soft tissue, according to claim 14, wherein the needle pushes fibers of the non-woven textile (34, 44) into the human or animal soft tissue.
